(19) Europäisches Patentamt
European Patent Office
Office européen des brevets

(11) Publication number: **0 273 188 B1**

(12) **EUROPEAN PATENT SPECIFICATION**

(45) Date of publication of patent specification: **10.06.92**

(51) Int. Cl.5: **C07J 31/00**, C07J 41/00, A61K 31/575

(21) Application number: **87117183.1**

(22) Date of filing: **21.11.87**

# (54) Process for preparing therapeutically active derivatives of ursodeoxycholic acid.

(30) Priority: **26.11.86 CH 4728/86**

(43) Date of publication of application: **06.07.88 Bulletin 88/27**

(45) Publication of the grant of the patent: **10.06.92 Bulletin 92/24**

(84) Designated Contracting States: **AT BE CH DE ES FR GB GR IT LI LU NL SE**

(56) References cited: **LU-A- 84 421**

(73) Proprietor: **Jago Research AG**
**Seestrasse 65**
**CH-6052 Hergiswil NW(CH)**

(72) Inventor: **Reiner, Alberto**
**Via Mentana 23**
**Como(IT)**

(74) Representative: **Frei, Alexandra Sarah**
**Frei Patentanwaltsbüro Hedwigsteig 6 Postfach 95**
**CH-8029 Zürich(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 273 188 B1

## Description

The present invention concerns a process for preparing ursodeoxycholic acid derivatives, more particularly thioesters, as well as the corresponding organic and inorganic salts with pharmaceutically acceptable bases.

The compounds to be prepared according to the present invention have the following, general, structural formula:

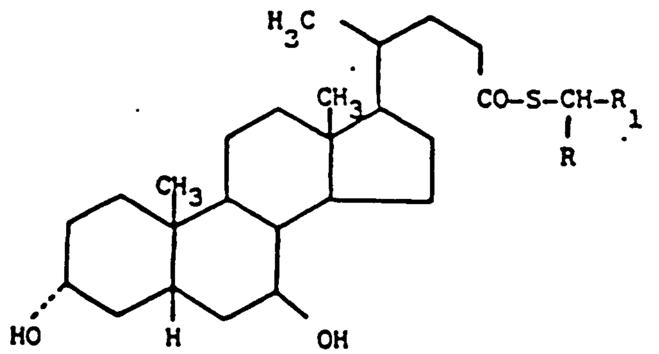

(I)

wherein R represents -H, $-CH_3$ or -COOH and $R_1$ represents $-CONHCH_2COOH$, $-CH_2COOH$ or

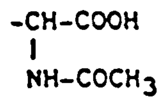

The derivatives prepared according to the present invention can be therapeutically employed mainly in the treatment of the altered biligenetic function, lithiasis or dyskinesia of the biliary ducts.

Are within the scope of the present process also the salts of thioesters of formula (1), either organic with aminoacids, such as lysine and arginine, or inorganic, such as the salts of sodium, calcium, etc.

It is object of the present invention the process for preparing derivatives of formula (1), characterized by the steps wherein:

a) the mixed anhydride of ursodeoxycholic acid with alkyl or phenyl chloroformate is prepared, and

b) the above mixed anhydride is esterified with a reactive compound, containing the -SH group, selected particularly from alfa-mercaptopropionylglycine and N-acetylcysteine.

In the first step of the process the mixed anhydride of ursodeoxycholic acid is prepared by reaction with an alkyl chloroformate, selected in particular from ethyl, butyl and isobutyl chloroformate, or with phenyl chloroformate, in the presence of an organic amine, particularly triethylamine, at temperature between 6° and 20° C.

The resulting mixed anhydride, in form of a white, heterogeneous material, is then reacted with the reactive compound containing the -SH group, at temperature below 10° C and in the presence of triethylamine.

When the addition of triethylamine has been completed, the temperature of the reaction mixture is allowed to spontaneously increase to about 29° C, owing to the low reaction exotherm.

The process according to the present invention will be now explained, only by way of illustration, by the following examples.

Example 1

Ursodeoxycholic acid thioester with alfa-mercaptopropionylglycine

($R = CH_3$; $R_1 = -CO-NHCH_2COOH$)

a) mixed anhydride of ursodeoxycholic acid.

To a solution of 19,6g of ursodeoxycholic acid in 80 ml of dioxane 4,75 ml of ethyl chlorformate are added and the solution is cooled to about 8° C in an ice bath.

1 Mole of triethylamine in dioxane (1:2,3 v/v) is slowly added, while maintaining the temperature between

8° and 10°C. A white, heterogeneous material, not very fluid, slowly develops, its fluidity increasing as the material is heated to room temperature (about 19°C.

b) To the material obtained in a) 9,8 g of alfa-mercaptopropionylglycine at room temperature are added, then the mixture is again cooled to about 8°C. A further 1 mole of triethylamine in dioxane is added.

This addition is carried out slowly at a temperature always below 10°C and, when it is completed, the temperature of the reaction mixture is allowed to increase spontaneously to about 19°C.

The completion of thioester formation reaction, which is a slow process requiring on the whole 7-10 hours, is slightly exothermic and consequently the temperature increases to about 29°C.

The reaction product is separated by dilution with 500 ml of aqueous HC1, extraction with chloroform and washing with water.

The chloroform phase is concentrated to a clear, colourless oil.

The successive purification of the above oil gives a white, microcrystalline power, m.p. 65-70°C (decomposes), soluble in cold alcohols and chloroform and insoluble in water and acetone.

UV, 1R and NMR spectra of the obtained product prove its structural formula.

Example 2

Ursodeoxycholic acid thioester with N-acetylcysteine

R= H 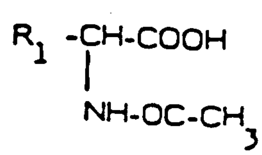

To the mixed anhydride of ursodeoxycholic acid, obtained according to the procedure of Example la starting form 10 g of acid, 4,5 g of N-acetylcysteine at 20°C are added and the reaction mixture is cooled to a temperature of 7°C.

1 Mole of triethylamine is then slowly added, while maintaining the temperature below 10°C.

When the addition has been completed, the reaction mixture is heated to 20°C and stirred up to completion of the reaction.

Following the procedure of Example 1, a product is obtained, in form of a white, microcrystalline powder, which decomposes at 57°C, but is totally liquid at 95° and soluble in cole clcohols, partially soluble in acetone and insoluble in water.

The product is chromatographically pure and the UV, 1R and NMR spectra prove its structure.

## Claims

**1.** Process for preparing thioesters of ursodeoxycholic acid of general formula:

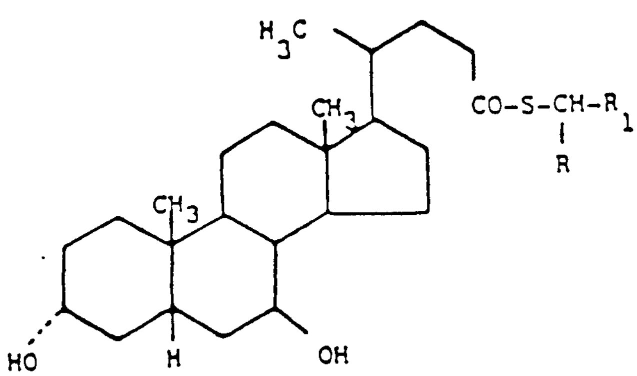

(I)

wherein R represents -H. $-CH_3$ or -COOH and $R_1$ represents $-CONHCH_2COOH$. $-CH_2COOH$ or

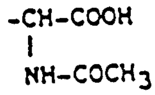

and their organic and inorganic, pharmaceutically acceptable salts,
characterized by the steps wherein:

a) the mixed anhydride of ursodeoxycholic acid with alkyl or phenyl chloroformate is prepared and
b) the above mixed anhydride is reacted with a reactive compound containing a -SH group.

**2.** Process according to claim 1 characterized in that said step (a) is carried out at a temperature not in excess of 10 ° C and in the presence of triethylamine.

**3.** Process according to claim 1. characterized in that said alkyl chloroformate is selected from ethyl. butyl and isobutyl chloroformate.

**4.** Process according to claim 1. characterized in that said step (a) is carried out in dioxane.

**5.** Process according to claim 1. characterized in that said step (b) is carried out by adding said reactive compound containing the -SH group and afterwards, slowly, triethylamine, the temperature during the addition being not in excess of 10 ° C.

**6.** Process according to claim 4. caracterized in that after the addition of triethylamine, the reaction mixture is maintained under stirring and its temperature is allowed to spontaneously increase.

**7.** Process according to claim 1 characterized in that said reactive compound containing the -SH group is selected from alfa-mercaptopropionylglycine and N-acetylcysteine.

**Revendications**

**1.** Procédé de préparation de thioesters d'acide ursodésoxycholique, représentés par la formule générale :

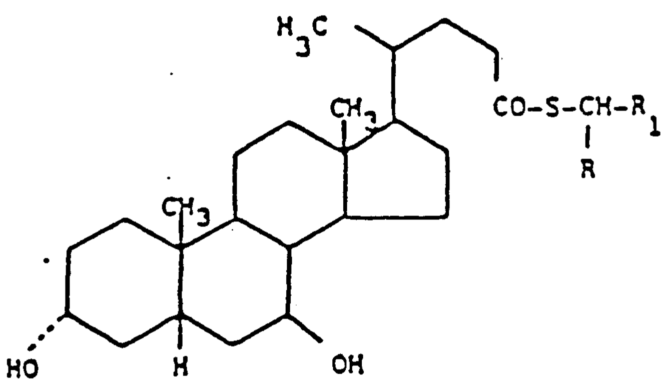

(I)

dans laquelle :

- R représente -H, $-CH_3$ ou -COOH ; et
- $R_1$ représente $-CONHCH_2COOH$, $-CH_2COOH$ ou

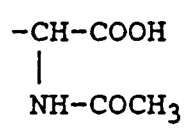

et leurs sels pharmaceutiquement acceptables, organiques et minéraux,
caractérisé par les étapes dans lesquelles :

(a) l'anhydride mixte de l'acide ursodésoxycholique avec un chloroformiate d'alkyle ou de phényle est préparé ; et
(b) l'anhydride mixte ci-dessus est mis à réagir avec un composé réactif contenant un groupe -SH.

**2.** Procédé selon la revendication 1, caractérisé par le fait que ladite étape (a) est effectuée à une température ne dépassant pas 10 ° C et en présence de triéthylamine.

**3.** Procédé selon la revendication 1, caractérisé par le fait que ledit chloroformiate d'alkyle est choisi parmi le chloroformiate d'éthyle, de butyle et d'isobutyle.

**4.** Procédé selon la revendication 1, caractérisé par le fait que ladite étape (a) est effectuée dans le dioxanne.

**5.** Procédé selon la revendication 1, caractérisé par le fait que ladite étape (b) est effectuée par addition dudit composé réactif contenant le groupe -SH et, ensuite, lentement, de triéthylamine, la température pendant l'addition ne dépassant pas 10 ° C.

**6.** Procédé selon la revendication 4, caractérisé par le fait gu'après l'addition de triéthylamine, le mélange réactionnel est maintenu sous agitation, et sa température est amenée à augmenter spontanément.

**7.** Procédé selon la revendication 1, caractérisé par le fait que ledit composé réactif contenant le groupe -SH est choisi parmi l'alpha-mercaptopropionylglycine et la N-acétylcystéine.

**Patentansprüche**

**1.** Verfahren zur Herstellung von Thioestern der Ursodesoxycholsäure mit der allgemeinen Strukturformel:

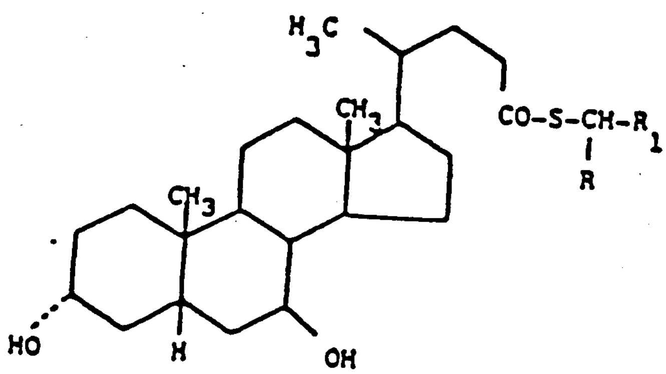

wobei R = -H, $CH_3$, oder -COOH
und $R_1$ = $-CONHCH_2COOH$, $-CH_2COOH$ oder

**-CH-COOH**
**$NH-COCH_3$**

und der entsprechenden organischen und anorganischen, pharmazeutisch akzeptablen Salze, **gekennzeichnet** durch die folgenden Schritte:

a) Herstellung eines gemischten Anhydrids der Ursodesoxycholsäure mit Alky- oder Phenyl-substituiertem Formylchlorid und
b) Reaktion des gemischten Anhydrids mit einer reaktiven Verbindung, die eine -SH Gruppe aufweist.

**2.** Verfahren nach Anspruch 1, **dadurch gekennzeichnet,** dass der erste Verfahrensschritt (a) bei einer Temperatur nicht über 10 ° C und in der Gegenwart von Triethylamin durchgeführt wird.

3. Verfahren nach Anspruch 1, **dadurch gekennzeichnet,** dass das EthylFormylchlorid Ethyl-, Butyl-, oder Isobutyl-Formylchlorid ist.

4. Verfahren nach Anspruch 1, **dadurch gekennzeichnet,** dass der erste Verfahrensschritt (a) in Dioxan durchgeführt wird.

5. Verfahren nach Anspruch 1, **dadurch gekennzeichnet,** dass im zwei ten Verfahrensschritt (b) die reaktive Verbindung mit der -SH Gruppe zugefügt wird und dann langsam Triethylamin zugefügt wird, wobei die Temperatur unterhalb von 10 ° C gehalten wird.

6. Verfahren nach Anspruch 4, **dadurch gekennzeichnet,** dass das Reaktionsgemisch nach dem Zufügen von Triethylamin gerührt wird, während es sich von selbst erwärmt.

7. Verfahren nach Anspruch 1, **dadurch gekennzeichnet,** dass die reaktive Verbindung mit der -SH Gruppe Alfa-Mercaptopropionylglycin oder N-Acetylcystein ist.